# EUROPEAN PATENT SPECIFICATION

(11) **EP 2 515 850 B1**
(45) Date of publication and mention of the grant of the patent: **29.06.2016**
(21) Application number: 10795487.7
(22) Date of filing: 03.12.2010
(51) Int. Cl.: A61K 9/16, A61K 9/20, A61K 31/545, A61K 9/46

(54) **PHARMACEUTICAL COMPOSITIONS COMPRISING CEFDINIR AS AN ACTIVE AGENT**
PHARMAZEUTISCHE ZUSAMMENSETZUNGEN MIT CEFDINIR ALS WIRKSTOFF
COMPOSITIONS PHARMACEUTIQUES COMPRENANT CEFDINIR COMME PRINCIPE

(30) Priority: 25.12.2009 TR 200909785
(43) Date of publication of application: 31.10.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000242
(87) International publication number: WO 2011/078822

(56) References cited:
- WO-A1-2004/104010
- DE-A1-102007 002 924
- US-A1- 2005 131 079
- US-A1- 2007 128 268
- US-A1- 2008 103 124
- DATABASE WPI Week 200630 Thomson Scientific, London, GB; AN 2006-285271 XP002620814, & CN 1 706 389 A (JINAN PINGZHI MEDICINE SCI TECH CO LTD) 14 December 2005 (2005-12-14)
- DATABASE WPI Week 200925 Thomson Scientific, London, GB; AN 2009-F19584 XP002620815, & CN 101 352 424 A (TIANJIN CENT MEDICINE CO LTD) 28 January 2009 (2009-01-28)

## Description

Present invention is related to water dispersible pharmaceutical dosage forms comprising cefdinir as active agent and methods for preparation of these.

### Background of the invention

Cefdinir molecule which is shown with Formula I was first disclosed in the patent numbered BE897864 and its chemical name is (6R,7R)-7-[[(2Z)-(2-amino-4-thiazolil)(hydroxyimino)acetyl]amino]-3-ethenyl-8-oxo-5-thia-1-azabicyclo[4.2.0]oct-2-en-2-carboxylic acid. The molecule which is a third generation cephalosporin, is indicated for the treatment of several illnesses caused by gram positive and gram negative bacteria.

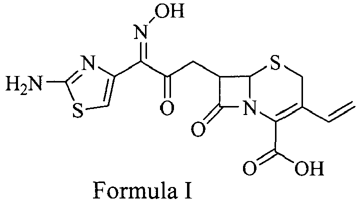

Cefdinir which physically appears as a white powder has very poor solubility in common organic solvents such as methanol, ethanol, and acetonitrile and in water. Due to this property there are some problems while developing formulations comprising this molecule and in the bioavailability of the finished product.

The product sold in the market under the tradename OMNICEF^{®} is present in capsule and suspension forms. Clinic studies show that the bioavailability of the suspension product is 120% more than the bioavailability of the product in capsule form.

Although the suspension forms have higher bioavailability, use of this dosage form especially for pediatric and geriatric patients brings about the possibility of taking high and/or uncontrolled dose. Additionally, the fact that the suspensions have physical and chemical stability problems, they have short shelf life and high production costs and the fact that they cause problems while transporting and use are disadvantageous for the manufacturers.

Due to the reasons stated above it is necessary to provide new dosage forms in antibiotic theraphy in order to provide effective dosing, meet patient requirements and to offer different alternatives to patients having special conditions, such as pediatric and geriatric patients.

The document WO2004/104010 discloses that cefdinir is soluble in alkaline solution comprising sodium bicarbonate. The document CN1706389 discloses cefdinir effervescent tablets comprising sodium bicarbonate.

When state of the art is taken into account it is seen that it is necessary to develop; new pharmaceutical compositions comprising cefdinir that are stable, have long shelf life, easy to use and that have high bioavailability and different dosage forms.

Inventors have surprisingly found that the problems present in the state of the art can be solved by the water dispersible powder, tablet, granule formulations prepared according to the present invention.

### Detailed description of the invention

Subject matter of the present invention is related to the cefdinir formulation as defined in claim 1 and to the process for preparation thereof. Surprisingly it was seen that when cefdinir, which is characterized with its low water solubility, is formulated with the water dispersible powder, tablet, granule formulation disclosed in the present invention it disperses in water and forms homogenous cefdinir solution. Accordingly, pharmaceutical dosage forms that are in the form of water dispersible powder, tablet or granule and suitable for use as a single dose;
a) Will be have a longer shelf life compared to the suspension forms since the dosage form in solid form is more stable and
b) Has higher bioavailability and is easier to use for the patients compared to the solid dosage forms since it dissolves in water and disperses homogeneously prior to use.

Therefore water dispersible powder, tablet and granule formulation of the present invention has combined the advantages of the tablet and suspension forms and removes the disadvantages arising from these forms.

The term "water dispersible powder, tablet and granule" comprises effervescent tableti effervescent granules, effervescent powders, water dispersible tablets, water dispersible powders and water dispersible granules, water soluble tablets, water soluble powders, water soluble granules.

Accordingly one aspect of the present invention is water dispersible powders, tablets and granules comprising cefdinir as active agent.

Another aspect of the invention is water dispersible powder, tablet and granule formulations comprising pharmaceutically acceptable excipients in addition to cefdinir which is used as an active agent.

Cefdinir has a hydrophobic character and for this reason it has wetting and low solubility problems. This leads to low bioavailability and problems related to development of water dispersible formulations.

Upon the investigations related to development of water dispersible powder, tablet and granule forms, inventors have unexpectedly found that use of organic base in the formulation is effective for solving the water solubility problem of cefdinir.

Organic base that is used in the formulation is selected from a group comprising ethanolamine, isopropanolamine, 1-deoxy-1-methylamino-sorbitol, 1-deoxy-1-methylamino-D-glucitol, tris(hydroxymethyl)aminomethane, N-(tri(hydroxymethyl)methyl)glycine, N,N-Bis(2-hydroxyethyl)glycine, 2-methyl aminophenol. Preferably 1-deoxy-1-methylamino-sorbitol and tris(hydroxymethyl)aminomethane are used.

Cefdinir which can be used in the water dispersible powder, tablet and granule formulations of the present invention can be present in the form of its solvates, hydrates, enetiomers, racemates, organic salts, inorganic salts, polymorphs, crystal and amorphous forms or in free form and/or as a combination of these.

In the said formulation in addition to cefdinir and organic base several other excipients such as binders, lubricants, humectants, disintegrants, basic agents, acidic agents, sweeteners and optionally effervescent couples can be used.

In water dispersible powder, tablet and granule formulation of the invention, binder can be selected from, but not limited with, a group comprising ethyl cellulose, gelatine, hydroxy ethyl cellulose, hydroxy methyl cellulose, hydroxypropyl cellulose, hypromellose, magnesium aluminium silicate, methyl cellulose, povidone.

In water dispersible powder, tablet and granule formulation of the invention, lubricant can be selected from, but not limited with, a group comprising calcium stearate, magnesium stearate, polyethylene glycol, PEG6000, polyvinyl alcohol, potassium benzoate, sodium benzoate.

In water dispersible powder, tablet and granule formulation of the invention, humectant can be selected from, but not limited with, a group comprising anhydrous sodium sulphate, silica gel and potassium carbonate.

In water dispersible powder, tablet and granule formulation of the invention, disintegrant can be selected from, but not limited with, a group comprising carboxymethyl cellulose calcium, carboxymethyl cellulose sodium, microcrystalline cellulose, silicon dioxide, croscarmellose sodium, crospovidone, hydroxypropyl cellulose, methyl cellulose, povidone, magnesium aluminium silicate, starch or a combination thereof.

In water dispersible powder, tablet and granule formulation of the invention, diluent can be selected from, but not limited with, a group comprising calcium carbonate, calcium sulfate, dibasic calcium phophate, tribasic calcium sulfate, calcium sulfate, microcrystalline cellulose, lactose, magnesium carbonate, magnesium oxide, maltodextrine, maltose, mannitol, sodium chloride, sorbitol, starch, xylitol or a combination thereof.

In water dispersible powder, tablet and granule formulation of the invention, basic agent can be selected from, but not limited with, a group comprising potassium carbonate, potassium citrate, potassium hydroxide, sodium carbonate, sodium bicarbonate or combinations thereof.

In water dispersible powder, tablet and granule formulation of the invention, acidic agent can be selected from, but not limited with, a group comprising acetic acid, citric acid, lactic acid, malic acid, phosphoric acid, propionic acid, tartaric acid or combinations thereof.

In water dispersible powder, tablet and granule formulation of the invention, sweetener can be selected from, but not limited with, a group comprising acesulfame, aspartamate, dextrose, fructose, glucose, lactitol, maltitol, maltose, sorbitol, saccharide, sodium saccharide, sodium cyclamate, sucralose, sodium chloride, potassium chloride, sucrose, xylitol or combinations thereof.

In water dispersible powder, tablet and granule formulation of the invention, effervescent couple which is optionally used can be selected from, but not limited with, a group comprising citric acid, tartaric acid, malic acid, furmaric acid etc as organic acid and sodium carbonate, sodium hydrogen carbonate, potassium carbonate, potassium hydrogen carbonate etc. as organic base.

In water dispersible powder, tablet and granule formulation of the invention, 1-4000 mg of cefdinir or pharmaceutical salts, hydrates, solvates or a combination thereof in an amount equivalent to that can be used.

In water dispersible powder, tablet and granule formulation of the invention; 5-60% of cefdinir or pharmaceutically acceptable solvates, hydrates, enantiomers, racemates, organic salts, inorganic salts, polymorphs, crystal/amorphous forms, 1-30% organic base, 1-30% binder, 0.1-3 % lubricant, % 0.1-5% sweetener, 0.1-8 % coloring and/or flavouring agent and optionally 0-90% effervescent couple in an amount by total weight of the unit dose can be used.

In another aspect present invention relates to processes used for the preperation of water dispersible powder, tablet and granule formulations comprising cefdinir as active agent and pharmaceutically acceptable excipients.

Accordingly, process used in present invention comprises granulation of cefdinir with conventional dry and/or wet granulation methods known in the art or mixing cefdinir and other excipients with a dry blending method and optionally pressing them in tablet form.

Water dispersible powder, tablet or granules according to the present invention can be prepared according to the examples given below. The examples are given for the sake of demonstrating the invention and the invention is not limited with these examples.

### EXAMPLE 1: Formulation and process for preparation of effervescent granules

| | % amount in unit dose | Amount in unit dose (mg) |
|---|---|---|
| Cefdinir | 30% | 600 mg |
| Organic base | 9% | 180 mg |
| Citric acid | 33% | 660 mg |
| Sodium hydrogen carbonate | 20% | 400 mg |
| Binder | 2.5% | 50 mg |
| Sweetener | 2.5% | 50 mg |
| Lubricant | 0.75% | 15 mg |
| Coloring agent | 1.25% | 25 mg |
| Flavouring agent | 1% | 20 mg |
| Total tablet weight | - | 2000 mg |

Formulation is obtained by granulation of sodium hydrogen carbonate and cefdinir with aqueous solution of organic base and then mixing the formed granules with citric acid and sweetener. The formed mixture is then granulated with a solution of binder. The granule obtained after this step is mixed with lubricant, coloring agent, sweetener and flavouring agent and optionally it can be compressed to obtain tablets.

### EXAMPLE 2: Formulation and process for preperation of water dispersible granules

| | % amount in unit dose | Amount in unit dose (mg) |
|---|---|---|
| Cefdinir | %45 | 450 mg |
| Organic base | %21 | 210 mg |
| Binder | %23 | 230 mg |
| Sweetener | %4.5 | 45 mg |
| Lubricant | %2 | 20 mg |
| Coloring agent | %2 | 20 mg |
| Flavouring agent | %2.5 | 25 mg |
| Total tablet weight | - | 1000 mg |

Formulation can be obtained by granulation of cefdinir with aqueous solution of organic base and then mixing the formed granules with sweetener. The formed mixture is then granulated with a solution of binder. The granule obtained after this step is mixed with lubricant, coloring agent, sweetener and flavouring agent and optionally it can be compressed to obtain tablets.

In another aspect present invention relates to use of water dispersible powder, tablet and granule formulations comprising cefdinir and in addition to that pharmaceutically acceptable excipients for the treatment of infections caused by gram positive and gram negative bacteria.

## Claims

1. A pharmaceutical composition comprising cefdinir and an organic base, wherein said composition is in effervescent powder, tablet and/or granule form; and wherein the organic base is selected from a group comprising ethanolamine, isopropanolamine, 1-deoxy-1-methylamino sorbitol, 1-deoxy-1-methylamino-D-glucitol, tris(hydroxymethyl)aminomethane, N-(tri(hydroxymethyl)methyl)glycine, N,N-Bis(2-hydroxyethyl)glycine and 2-methyl aminophenol.

2. The pharmaceutical composition according to claim 1 wherein the organic base is 1-deoxy-1-methylamino-sorbitol and/or tris(hydroxymethyl)aminoethane.

3. The composition according to claim 1 wherein cefdinir can be in form of its solvates, hydrates, enantiomers, racemates, organic salts, inorganic salts, polymorphs, crystal and amorphous forms or in free form and/or as a combination of these.

4. The composition according to claim 1 wherein said composition comprises pharmaceutically acceptable excipients in addition to cefdinir that is used as an active agent, preferably wherein the pharmaceutically acceptable excipients are binders, lubricants, humectants, diluents, disintegrants, basis agents, sweeteners and optionally effervescent agents.

5. The pharmaceutical composition according to claim 4, wherein binder can be selected from a group comprising ethyl cellulose, gelatine, hydroxyethyl cellulose, hydroxymethyl cellulose, hydroxypropyl cellulose, hypromellose, magnesium aluminium silicate, methyl cellulose, povidone.

6. The pharmaceutical composition according to claim 4 wherein lubricant can be selected from a group comprising calcium stearate, magnesium stearate, polyethylene glycol, PEG 6000, polyvinyl alcohol, potassium benzoate, sodium benzoate.

7. The pharmaceutical composition according to claim 4 wherein humectant can be selected from a group comprising sodium sulfate, silica gel, potassium carbonate.

8. The pharmaceutical composition according to claim 4 wherein disintegrant can be selected from a group comprising carboxymethyl cellulose calcium, carboxymethyl cellulose sodium, microcrystalline cellulose, silicon dioxide, croscarmellose sodium, crospovidon, hydroxypropylcellulose, methyl cellulose, povidone, magnesium aluminium silicate, starch or a combination thereof.

9. The pharmaceutical composition according to claim 4 wherein diluent can be selected from a group comprising calcium carbonate, calcium sulfate, dibasic calcium phosphate, tribasic calcium phosphate, calcium sulfate, microcrystalline cellulose, lactose, magnesium carbonate, magnesium oxide, maltodextrin, maltoz, mannitol, sodium chloride, sorbitol, starch, xylitol or combinations thereof.

10. The pharmaceutical composition according to claim 4 wherein basic agent can be selected from a group comprising potassium carbonate, potassium bicarbonate, potassium citrate, potassium hydroxide, sodium carbonate, sodium bicarbonate, or combinations thereof; and
wherein acidic agent can be selected from a group comprising acetic acid, citric acid, lactic acid, malic acid, phosphoric acid, propionic acid, tartaric acid or combinations thereof.

11. The pharmaceutical composition according to claim 4 wherein sweetener can be selected from a group comprising acesulfame, aspartamate, dextrose, fructose, glucose, lactitol, maltitol, maltose, sorbitol, saccharine, saccharine sodium, sodium cyclamate, sucralose, xylitol, sodium chloride, potassium chloride or combinations thereof.

12. The pharmaceutical composition according to claim 1-11 wherein compared to the total weight of the unit dose; 5-60% of cefdinir or pharmaceutically acceptable solvates, hydrates, enantiomers, racemates, organic salts, inorganic salts, polymorphs, crystal/amorphous forms, 1-30% organic base, 1-30% binder, 0.1-3 % lubricant, % 0.1-5% sweetener, 0.1-8 % coloring and/or flavouring agent and optionally 0-90% effervescent couple can be used.

13. A process for the preparation of the formulations according to claims 1-12 wherein said process comprises granulation of cefdinir with an aqueous solution of the organic base under dry and/or wet granulation methods or by mixing cefdinir and other excipients with a dry blending method and optionally pressing them in tablet form.

14. The composition according to any of claims 1 to 13 for use in the treatment of infections caused by gram positive and gram negative bacteria.

## Patentansprüche

1. Pharmazeutische Zusammensetzung, die Cefdinir und eine organische Base beinhaltet, wobei die erwähnte Zusammensetzung in Form von Brausepulver , Tablette und / oder Granulat ist; und wobei die organische Base aus einer Gruppe ausgewählt wird, die Ethanolamin, Isopropanolamin, 1-deoxy-1-methylamino Sorbitol, 1-deoxy-1-methylamino-D-Glucitol, Tris (hydroxymethyl) aminomethan, N- (tri (hydroxymethyl)methyl) Glycin, N,N-Bis (2-hydroxyethyl) glycin und 2-Methyl-aminophenol beinhaltet.

2. Pharmazeutische Zusammensetzung nach Anspruch 1, wobei die organische Base 1-deoxy-1-methylamino-Sorbit und / oder Tris (hydroxymethyl) aminomethan ist.

3. Die Zusammensetzung nach Anspruch 1, wobei sich Cefdinir in Form von seinen Solvaten , Hydraten, Enantiomeren, Racematen, organischen Salzen, anorganischen Salzen, Polymorphen, in den Kristall- und amorphen Formen oder in freier Form und / oder als eine Kombination von diesen befinden kann.

4. Zusammensetzung nach Anspruch 1, wobei die erwähnte Zusammensetzung pharmazeutisch annehmbare Hilfsstoffe zusätzlich zu dem Cefdinir beinhaltet, das als aktives Mittel verwendet wird, wobei vorzugsweise die pharmazeutisch annehmbaren Hilfsstoffe Bindemittel, Gleitmittel, Feuchthaltemittel, Verdünnungsmittel, Sprengmittel , Basismittel, Süßungsmittel und gegebenenfalls Brausemittel sind.

5. Pharmazeutische Zusammensetzung nach Anspruch 4, wobei Bindemittel aus einer Gruppe ausgewählt wird, die Ethylcellulose, Gelatine, Hydroxyethylcellulose, Hydroxymethylcellulose, Hydroxypropylcellulose, Hypromellose, Magnesiumaluminiumsilikat , Methylcellulose und Povidon beinhaltet.

6. Pharmazeutische Zusammensetzung nach Anspruch 4, wobei Gleitmittel aus einer Gruppe ausgewählt wird, die Calciumstearat, Magnesiumstearat, Polyethylenglykol , PEG 6000, Polyvinylalkohol, Kaliumbenzoat und Natriumbenzoat beinhaltet.

7. Pharmazeutische Zusammensetzung nach Anspruch 4, wobei Feuchthaltemittel aus einer Gruppe ausgewählt werden können, die Natriumsulfat, Kieselgel und Kaliumcarbonat beinhaltet.

8. Pharmazeutische Zusammensetzung nach Anspruch 4, wobei Sprengmittel aus einer Gruppe ausgewählt werden können, die Carboxymethylcellulose-Calcium, Carboxymethylcellulose-Natrium, mikrokristalline Cellulose, Siliciumdioxid, Croscarmellose-Natrium, Crospovidon, Hydroxypropylcellulose, Methylcellulose, Povidon, Magnesiumaluminiumsilikat, Stärke oder eine Kombination davon beinhaltet.

9. Pharmazeutische Zusammensetzung nach Anspruch 4, wobei Verdünnungsmittel aus einer Gruppe ausgewählt werden können, die Calciumcarbonat, Calciumsulfat, dibasisches Calciumphosphat, tribasisches Calciumphosphat, Calciumsulfat, mikrokristalline Cellulose, Lactose, Magnesiumcarbonat, Magnesiumoxid, Maltodextrin, Maltose, Mannit, Natriumchlorid, Sorbit, Stärke, Xylitol oder Kombinationen davon beinhaltet.

10. Pharmazeutische Zusammensetzung nach Anspruch 4, wobei basisches Mittel aus einer Gruppe ausgewählt werden kann, die Kaliumcarbonat, Kaliumbicarbonat, Kaliumcitrat, Kaliumhydroxid, Natriumcarbonat, Natriumhydrogencarbonat, oder Kombinationen davon beinhaltet; und
wobei saures Mittel aus einer Gruppe ausgewählt werden kann, die Essigsäure, Citronensäure, Milchsäure, Apfelsäure, Phosphorsäure, Propionsäure, Weinsäure oder Kombinationen davon beinhaltet.

11. Pharmazeutische Zusammensetzung nach Anspruch 4, wobei Süßungsmittel aus einer Gruppe ausgewählt werden können, die Acesulfam, Aspartam, Dextrose, Fructose, Glucose, Lactitol, Maltitol, Maltose, Sorbit, Saccharin, Saccharin-Natrium, Natrium-Cyclamat, Sucralose, Xylit, Natriumchlorid, Kalium Chlorid oder Kombinationen davon beinhaltet.

12. Pharmazeutische Zusammensetzung nach Anspruch 1-11, wobei im Vergleich zu dem Gesamtgewicht der Einheitsdosis; 5-60% von Cefdinir oder pharmazeutisch annehmbaren Solvaten, Hydraten, Enantiomeren, Racematen, organischen Salzen, anorganischen Salzen, Polymorphen, crystal / amorphen Formen, 1-30% der organischen Base, 1-30% vom Bindemittel, 0.1-3% vom Gleitmittel, 0.1-5% vom Süßungsmittel, 0.1-8% vom Farb- und / oder Aromamittel und gegebenenfalls 0-90% vom Brausepaar verwendet werden können.

13. Ein Verfahren zur Herstellung der Formulierungen nach den Ansprüchen 1-12, wobei das erwähnte Verfahren die Granulierung vom Cefdinir mit einer wässrigen Lösung der organischen Base unter trockenen und / oder nassen Granulierungsverfahren oder das Mischen des Cefdinirs und der anderen Hilfsstoffe mit einem Trockenmischverfahren und gegebenenfalls das Pressen in Tablettenform umfasst.

14. Zusammensetzung nach einem der Ansprüche 1 bis 13 zur Verwendung bei der Behandlung von durch grampositive und gramnegative Bakterien verursachten Infektionen.

## Revendications

1. La composition pharmaceutique comprenant cefdinir et une base organique, dans laquelle ladite composition est en forme de poudre effervescente, comprimé et / ou sous forme de granules; et dans lequel la base organique est choisie d' un groupe comprenant l'éthanolamine, l'isopropanolamine, 1-désoxy-1-méthylamino le sorbitol, le 1-désoxy- 1-diméthylamino-D-glucitol, le tris (hydroxyméthyl) aminométhane, le N-(tri (hydroxyméthyl) méthyl) glycine, N, N-Bis (2-hydroxyéthyl) glycine et du 2- aminophénol de méthyle.

2. La composition pharmaceutique selon la revendication 1, dans lequel la base organique est de 1-désoxy-1-méthylamino-sorbitol et / ou le tris (hydroxyméthyl) aminométhane.

3. La composition selon la revendication 1, dans lequel le cefdinir peut être sous forme de ses produits de solvatation, des hydrates, énantiomères, racémates, sels organiques, des sels inorganiques, des polymorphes, des formes cristalline et amorphe ou sous forme libre et / ou sous forme d'une combinaison de ceux-ci.

4. La composition selon la revendication 1, dans laquelle ladite composition comprend des excipients pharmaceutiquement acceptables, en plus de Cefdinir qui est utilisé en tant qu'agent actif, de préférence dans laquelle les excipients pharmaceutiquement acceptables sont des liants, des lubrifiants, des agents humectants, des diluants, des désintégrants, des agents de base, des édulcorants et le cas échéant les agents effervescents.

5. La composition pharmaceutique selon la revendication 4, dans lequel un liant peut être choisi dans un groupe comprenant l'éthylcellulose, la gélatine, l'hydroxyéthylcellulose, l'hydroxyméthylcellulose, l'hydroxypropylcellulose, l'hypromellose, le silicate de magnésium aluminium, la méthylcellulose, la povidone.

6. La composition pharmaceutique selon la revendication 4, dans lequel le lubrifiant peut être choisi dans un groupe comprenant le stéarate de calcium, le stéarate de magnésium, le polyethylene glycol, le PEG 6000, l'alcool de polyvinyle, le benzoate de potassium, benzoate de sodium.

7. La composition pharmaceutique selon la revendication 4, dans lequel humectant peut être choisi parmi un groupe comprenant du sulfate de sodium, le gel de silice, le carbonate de potassium.

8. La composition pharmaceutique selon la revendication 4, dans lequel délitant peut être choisi parmi un groupe comprenant la carboxyméthylcellulose calcique, la carboxyméthylcellulose sodique, la cellulose microcristalline, le dioxyde de silicium, croscarmellose sodique, la crospovidone, l'hydroxypropylcellulose, la méthylcellulose, la povidone, le silicate de magnésium et d'aluminium, de l'amidon ou une combinaison de celui-ci.

9. La composition pharmaceutique selon la revendication 4, dans lequel le diluant peut être choisi dans un groupe comprenant le carbonate de calcium, le sulfate de calcium, le phosphate de calcium dibasique, le phosphate de calcium tribasique, le sulfate de calcium, la cellulose microcristalline, le lactose, le carbonate de magnésium, l'oxyde de magnésium, la maltodextrine, le maltose, le mannitol, le chlorure de sodium, le sorbitol, l'amidon, le xylitol ou une combinaison de ceux-ci.

10. La composition pharmaceutique selon la revendication 4, dans lequel l'agent basique peut être choisi dans un groupe comprenant le carbonate de potassium, le bicarbonate de potassium, le citrate de potassium, l'hydroxyde de potassium, le carbonate de sodium, bicarbonate de sodium, ou des combinaisons de ceux-ci; et y est un agent acide peut être choisi dans un groupe comprenant l'acide acétique, l'acide citrique, l'acide lactique, l'acide malique, l'acide phosphorique, l'acide propionique, l'acide tartrique ou des combinaisons de ceux-ci.

11. La composition pharmaceutique selon la revendication 4, dans lequel l'édulcorant peut être choisi dans un groupe comprenant l'acésulfame, l'aspartame, le dextrose, le fructose, le glucose, le lactitol, le maltitol, le maltose, le sorbitol, la saccharine, la saccharine sodique, le cyclamate de sodium, le sucralose, le xylitol, le chlorure de sodium, de potassium le chlorure ou une combinaison de ceux-ci.

12. La composition pharmaceutique selon la revendication 1-11, dans lequel par rapport au poids total de la dose unitaire; 5-60% de cefdinir ou solvates pharmaceutiquement acceptables, leurs hydrates, énantiomères, racémates, sels organiques, des sels inorganiques, des cristaux polymorphes, formes amorphes, / 1-30% d'une base organique, de 1-30% de liant, 0.1-3% De lubrifiant, 0.1-5% d'édulcorant, de 0.1-8 % colorant et / ou un agent aromatisant et, facultativement, de 0-90% en couple effervescent peut être utilisé.

13. Un procédé pour la préparation des formulations selon les revendications 1-12, dans lequel ledit procédé comprend la granulation de cefdinir avec une solution aqueuse de la base organique dans des procédés de granulation par voie sèche et / ou humide ou par mélange cefdinir et d'autres excipients avec un mélange à sec méthode et éventuellement en les pressant sous forme de comprimés.

14. La composition selon l'une quelconque des revendications 1 à 13 pour une utilisation dans le traitement d'infections causées par des bactéries gram négatives et gram positives.
